**Europäisches Patentamt**

**European Patent Office**

**Office européen des brevets**

(19)

(11) Veröffentlichungsnummer: **0 211 146 B1**

(12) **EUROPÄISCHE PATENTSCHRIFT**

(45) Veröffentlichungstag der Patentschrift:
**08.11.89**

(51) Int. Cl.⁴: **A61N 1/22**, A61N 1/04, A61N 1/26

(21) Anmeldenummer: **86105149.8**

(22) Anmeldetag: **15.04.86**

(54) **Vorrichtung zur elektrischen Behandlung von Körperteilen.**

(30) Priorität: **19.04.85 DE 3514210**

(43) Veröffentlichungstag der Anmeldung:
**25.02.87 Patentblatt 87/9**

(45) Bekanntmachung des Hinweises auf die Patenterteilung:
**08.11.89 Patentblatt 89/45**

(84) Benannte Vertragsstaaten:
**AT BE CH DE FR GB IT LI LU NL SE**

(56) Entgegenhaltungen:
**EP-A- 0 128 103**
**AT-B- 273 359**
**DE-A- 2 906 103**
**FR-A- 2 149 684**
**US-A- 3 741 203**

**Auszüge aus den Gebrauchsmustern, Ausgabe A,
Heft 40, 6. Oktober 1983, München, Seiten 1935, 1936,
DE-U-8 310 564**

(73) Patentinhaber: **Kopetzky, Claus-Dieter, Dr.med.,
Provinzialstrasse 10,
D-6633 Wadgassen-Schaffhausen(DE)**

(72) Erfinder: **Kopetzky, Claus-Dieter, Dr.med.,
Provinzialstrasse 10,
D-6633 Wadgassen-Schaffhausen(DE)**

(74) Vertreter: **Schmidt, Hans-Ekhardt, Dipl.-Ing., Robert
Bosch GmbH Geschäftsbereich Mobile Kommunikation
Patent- und Lizenzabteilung Forckenbeckstrasse 9-13,
D-1000 Berlin 33(DE)**

ACTORUM AG

## Beschreibung

Die Erfindung betrifft eine Vorrichtung zur elektrischen Behandlung von Körperteilen, bestehend aus einem Paßteil, das bis auf mindestens eine Öffnung verschließbar und damit an die Form des zu behandelnden Körperteils anpaßbar ist, wobei die Innenwandung des Paßteils nachgiebig ist und mindestens zwei großflächige, aus leitfähigem Material bestehende und als Folien ausgebildete Elektroden trägt, die unter Druck dicht an das zu behandelnde Körperteil anlegbar und über Verbindungsleitungen mit einem Reizstromgerät verbindbar sind.

Eine derartige Vorrichtung ist durch die DE-OS 26 18 947 bekannt. Bei dieser bekannten Vorrichtung ist das Paßteil im Bereich der Außenwand ziemlich starr, so daß je nach Form und Größe des zu behandelnden Körperteils eine Vielzahl von Paßteilen bereitgehalten werden muß. Darüber hinaus ist mit dieser bekannten Vorrichtung nur bedingt eine Kompressionsbehandlung des vom Paßteil aufgenommenen Körperteils möglich.

Aus der AT-PS 131 791 ist es bekannt, eine Reizstrombehandlung mit einer Vakuumbehandlung zu kombinieren, um dadurch eine unterstützende Wirkung bei erhöhtem Behandlungseffekt zu erhalten. Bei der Vakuumbehandlung wird im zu behandelnden Körperteil der Zustand kräftiger Durchblutung hergestellt, bei dem die Reizstrombehandlung effektiver ist. Dazu sind aber auf das zu behandelnde Körperteil abgestimmte evakuierbare Vorrichtungen, wie Hauben oder Glocken nötig.

Aus dem DE-GM 83 10 564 ist eine Vorrichtung zur elektrischen und Magnetfeld-Behandlung von Körperteilen bekannt, bestehend aus einem Paßteil, das als flexible Manschette ausgebildet ist, die bis auf mindestens eine Öffnung verschließbar und damit an die Form des zu behandelnden Körperteils anpaßbar ist. Das Paßteil, das unter Druck dicht an das Körperteil anlegbar und über Verbindungsleitungen mit einem Reizstromgerät verbindbar ist, trägt an seiner Innenwandung mehrere im Abstand voneinander angeordnete, großflächige Elektroden, die aus leitfähigem Material bestehen und als Folien ausgebildet sind. Diese bekannte Vorrichtung ermöglicht eine zeitlich aufeinanderfolgende Aktivierung der Elektroden und damit eine Durchflutung des zu behandelnden Körperteils mit Reizstromimpulsen in den verschiedensten Richtungen.

Es ist Aufgabe der Erfindung, eine Vorrichtung der eingangs erwähnten Art zu schaffen, mit der neben der elektrischen Behandlung auch eine Kompressionsbehandlung des vom Paßteil aufgenommenen Körperteils möglich ist, wobei sich das Paßteil in weit größerem Maße an die Form und Größe des zu behandelnden Körperteils anpassen kann und die elektrische Behandlung in Verbindung mit der Kompressionsbehandlung wirksamer gestaltet werden kann.

Diese Aufgabe wird nach der Erfindung dadurch gelöst, daß das Paßteil als doppelwandige, aufblasbare und flexible Manschette ausgebildet und mittels eines Anschlusses mit einer Pumpe verbindbar ist, und daß die zwei Elektroden und mindestens zwei weitere Elektroden in Richtung quer zu einer Längsrichtung der Manschette im Abstand nebeneinander angeordnet sind.

Durch die Ausbildung des Paßteiles als flächige, doppelwandige und aufblasbare Manschette, die in ihrer Länge und lichten Weite auf die Abmessungen eines zu behandelnden Körperteils abgestimmt und mittels eines Anschlusses mit einer Pumpe verbindbar ist, wird nicht nur eine bessere Anpassung des Paßteiles an die Form und Größe des zu behandelnden Körperteils erreicht, die Vorrichtung ist dadurch auch für eine kombinierte Elektro- und Kompressionsbehandlung einsetzbar. Dabei bleiben die Möglichkeiten einer individuellen Elektro- oder Kompressionsbehandlung unverändert aufrechterhalten. Das Vorsehen mehrerer Elektrodenpaare in Längsrichtung der Manschette bietet den Vorteil, daß nicht nur eine großflächige Behandlung möglich ist, es lassen sich die Elektrodenpaare in einfacher Weise auch in beliebiger Reihenfolge aktivieren. Durch die gleichzeitige Anwendung des Reizstromes mit der Kompressionsbehandlung wird synergistisch sowohl eine Entspeicherung des Unterhautgewebes als auch der kapillaren Endstrombahnen der Muskulatur erreicht, was durch die Aktivierung der Elektrodenpaare in Richtung zum Herz noch unterstützt wird.

Vorteilhafte Weiterbildungen der Erfindung sind Gegenstand der Unteransprüche.

Das Anlegen der Manschette an das zu behandelnde Körperteil wird nach einer Ausbildung dadurch erleichtert, daß die Manschette entlang ihrer Längsrichtung einen Reißverschluß oder Klettverschluß aufweist.

Für die einfache Anbringung des Reizstromgerätes ist nach einer Ausgestaltung vorgesehen, daß die Manschette auf ihrer Außenwandung eine Halterung zum Befestigen des Reizstromgerätes aufweist.

Die Verwendung der Manschette in geöffnetem Zustand zur Behandlung des Körperstammes wird dadurch verbessert, daß die geöffnete Manschette etwa T-Form hat, wobei der Liegekomfort im Bereich des Nackens dadurch erhöht wird, daß die Manschette in der Mitte ihres durch die T-Form gegebenen breiteren Abschnittes eine in ihrer Längsrichtung verlaufende, muldenartige Vertiefung aufweist.

Nach einer weiteren Ausgestaltung ist vorgesehen, daß die Verbindungsleitungen vom Reizstromgerät zu den Elektroden im Inneren der Manschette verlaufend angeordnet sind, damit die Verbindungsleitungen zu den Elektroden die Verwendung der Manschette in dem geschlossenen oder geöffneten Zustand nicht beeinträchtigen.

Die Erfindung wird anhand eines in den Zeichnungen dargestellten Ausführungsbeispiels näher erläutert. Es zeigt:

Fig. 1 eine Vorrichtung in geschlossenem Zustand,

Fig. 2 die Vorrichtung nach Fig. 1 in geöffnetem Zustand,

Fig. 3 die Befestigung einer Reizstromquelle an der Außenwandung in vergrößerter Darstellung im Detail und

Fig. 4 den breiteren Abschnitt der Vorrichtung im Schnitt nach IV-IV der Fig. 2.

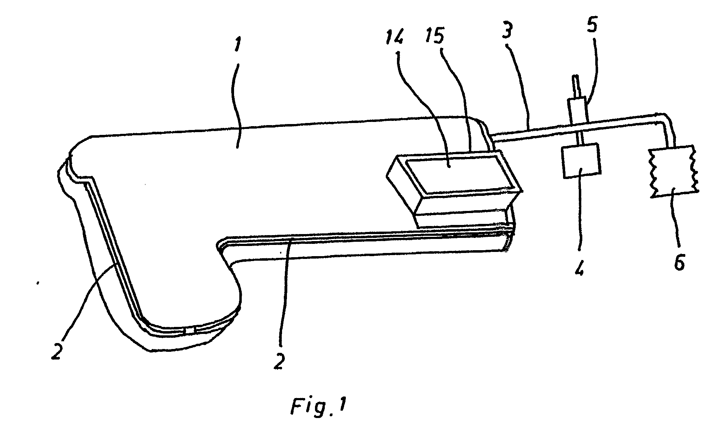

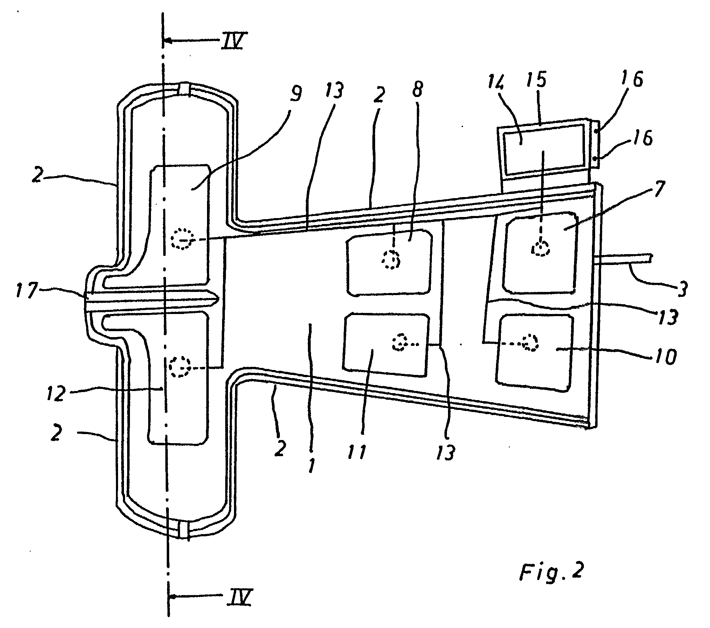

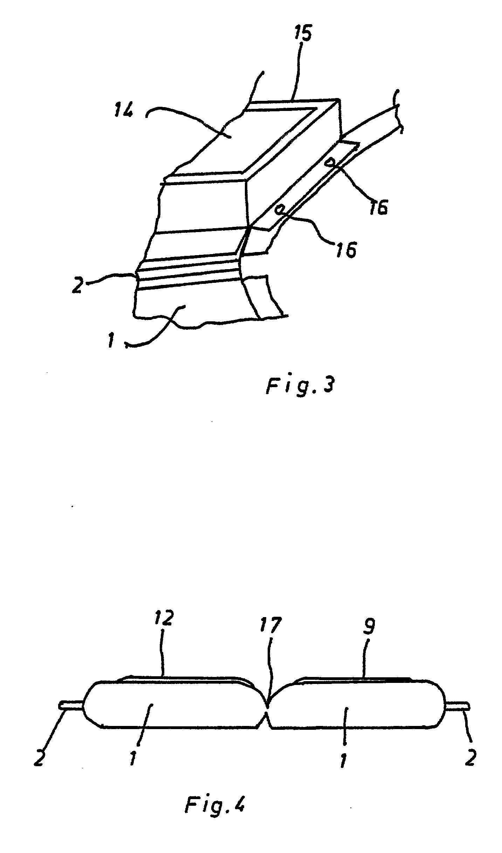

Nach den Fig. 1 bis 4 besteht eine Vorrichtung zur kombinierten Kompressions- und Reizstrombehandlung aus einer einseitig offenen, doppelwandigen, aufblasbaren Manschette 1, deren Abmessungen den maximalen Abmessungen der zu behandelnden Extremität angepaßt sind. Wie die Fig. 2 zeigt, hat die geöffnete Manschette im wesentlichen T-Form. Die Manschette ist an ihren Längsrändern, sowie an einem Ende mit Reißverschlüssen 2 oder Klettverschlüssen versehen, so daß die Manschette 1 geschlossen werden kann.

Wie Fig. 1 zeigt, ist mit der Manschette 1 ein Anschluß 3 mit einem Manometer 4 und einem Überdruckventil 5 verbunden. Der Anschluß 3 ist mit einer Pumpe 6 verbunden, durch die der Raum zwischen den Wandungen der Manschette 1 aufgeblasen werden kann.

Wie die Fig. 2 zeigt, sind an der Innenwandung der Manschette 1 im Abstand zueinander Elektroden 7 bis 12 angeordnet, die durch Leitungen 13, die im Inneren der Manschette 1 verlaufen, mit einem an der Außenseite der Manschette 1 angeordneten Reizstromgerät 14 verbunden sind.

Wie Fig. 3 zeigt, ist das Reizstromgerät 14 in einer Halterung 15 der Manschette 1 angeordnet, wobei die Halterung 15 durch Druckknöpfe 16 an der Manschette 1 im geschlossenen Zustand fixiert werden kann. Hierdurch entfallen alle störenden und freiliegenden Kabel. Die zu den Elektroden 7 bis 12 führenden Verbindungsleitungen 13 sind verdeckt verlegt. Bei geöffneter Manschette kann das Reizstromgerät 14 mit der Halterung 15 durch Lösen der Druckknöpfe 16 seitlich von der Manschette 1 abgeklappt werden, wie dies Fig. 2 zeigt.

Wie Fig. 2 und 4 zeigen, weist die Manschette 1 in der Mitte ihres breiteren Abschnittes eine in Längsrichtung der Manschette 1 verlaufende, muldenartige Vertiefung 17 auf.

Die Elektroden 7 bis 12 bestehen aus leitfähigen Folien, die mit der Innenwandung der Manschette 1 flächenbündig verbunden sind, vgl. Fig. 4 mit den nebeneinander angeordneten Elektroden 9 und 12.

Bei Anwendung der Vorrichtung wird die Extremität in der Manschette 1 gelagert und durch Schließen der Reißverschlüsse 2 die Manschette 1 geschlossen. Anschließend wird die Manschette 1 mittels der Pumpe 6 aufgepumpt, so daß sich die Innenwandung der Manschette 1 und damit die Elektroden 7 bis 12 einfach, schnell, gleichmäßig und sanft an die entsprechende Extremität anlegen. Dies ist unabhängig von der jeweiligen Form oder Größe der umschlossenen Extremität gewährleistet, solange die Maße der äußeren Manschettenhülle nicht überschritten werden.

Die Gleichmäßigkeit des Andrucks über die Gesamtfläche der Elektroden 7 bis 12 vermeidet das Auftreten unangenehmer Hautreizungen auch bei größeren Stromstärken.

Durch den gleichmäßigen Druck der Manschette 1 einerseits und den zunehmenden Durchmesser der Extremität nach proximal andererseits, ergibt sich ein von peripher nach proximal abnehmender Druckgradient im Inneren der Extremität. Dies unterstützt auf physiologische Art und Weise den lymphatischen und venösen Rückstrom. Wesentlich ist, daß durch die gleichzeitige Anwendung des Reizstromes zusammen mit dem Druck der Manschette 1 synergistisch eine Entspeicherung des Unterhautgewebes und der kapillaren Endstrombahn der Muskulatur erreicht wird.

Bei der Behandlung von arteriellen Durchblutungsstörungen wird eine übermäßige Kompression vermieden. Der Manschettendruck wird dann so gewählt, daß ein ausreichender Hautkontakt mit den Elektroden gewährleistet ist.

Zur Anwendung der Vorrichtung im Bereich des Körperstammes kann die Manschette 1 in geöffnetem Zustand verwendet werden.

Dazu wird die Manschette 1 ausreichend mit Luft gefüllt und der Patient auf ihr gelagert, was ein sicheres Anliegen der Elektroden 7 bis 12 gewährleistet. Die muldenförmige Vertiefung 17 im breiteren Abschnitt der Manschette 1 sichert in Rückenlage eine gleichmäßige Anlage der Elektroden im Nackenbereich.

Eine Korrektur der Elektrodenplazierung 7 bis 12 ist nicht nur durch eine veränderte Lagerung des Patienten möglich, es können auch Elektroden teilweise oder ganz abgedeckt werden, oder auf die Elektroden z.B. feuchte Tücher gelegt werden.

**Patentansprüche**

1. Vorrichtung zur elektrischen Behandlung von Körperteilen, bestehend aus einem Paßteil, das bis auf mindestens eine Öffnung verschließbar und damit an die Form des zu behandelnden Körperteils anpaßbar ist, wobei die Innenwandung des Paßteils nachgiebig ist und mindestens zwei großflächige, aus leitfähigem Material bestehende und als Folien ausgebildete Elektroden trägt, die unter Druck dicht an das zu behandelnde Körperteil anlegbar und über Verbindungsleitungen mit einem Reizstromgerät verbindbar sind, dadurch gekennzeichnet, daß das Paßteil als doppelwandige, aufblasbare und flexible Manschette (1) ausgebildet und mittels eines Anschlusses (3) mit einer Pumpe (6) verbindbar ist, und daß die zwei Elektroden (7,10) und mindestens zwei weitere Elektroden (8,11 bzw. 9,12) in Richtung quer zu einer Längsrichtung der Manschette (1) im Abstand nebeneinander angeordnet sind.

2. Vorrichtung nach Anspruch 1, dadurch gekennzeichnet, daß die Manschette (1) entlang ihrer Längsrichtung einen Reißverschluß (2) oder Klettverschluß aufweist.

3. Vorrichtung nach Anspruch 1 oder 2, dadurch gekennzeichnet, daß die Manschette (1) auf ihrer Außenwandung eine Halterung (15) zum Befestigen des Reizstromgerätes (14) aufweist.

4. Vorrichtung nach einem der Ansprüche 1 bis 3, dadurch gekennzeichnet, daß die geöffnete Manschette (1) etwa T-Form hat.

5. Vorrichtung nach Anspruch 4, dadurch gekennzeichnet, daß die Manschette (1) in der Mitte ihres durch die T-Form gegebenen breiteren Abschnittes eine in ihrer Längsrichtung verlaufende, muldenartige Vertiefung (17) aufweist.

6. Vorrichtung nach einem der Ansprüche 1 bis 5, dadurch gekennzeichnet, daß die Verbindungsleitungen (13) vom Reizstromgerät (14) zu den Elektroden (7 bis 12) im Inneren der Manschette (1) verlaufend angeordnet sind.

## Claims

1. A device for the electrical treatment of parts of the body, comprising a fitting part which is closable with the exception of at least one opening and is thus adaptable to the shape of the part of the body to be treated, the inner wall of which fitting part is flexible and carries at least two large-area electrodes which are made from concuctive material and are in the form of films and can be applied under pressure to the part of the body to be treated and are connectible to a stimulation current unit by way of connection leads, characterized in that the fitting part is in the form of a double-walled, inflatable and flexible sleeve (1) and is connectible to a pump (6) by way of a connection (3), and that the two electrodes (17, 20) and at least two further electrodes (8, 11 or 9, 12) are disposed adjacent to one another and spaced apart in a direction transversely to a longitudinal direction of the sleeve (1).

2. A device as claimed in claim 1, characterized in that the sleeve (1) has a zip fastener (2) or velcro fastener extending along the longitudinal direction thereof.

3. A device as claimed in claim 1 or 2, characterized in that the outer wall of the sleeve (1) has a holder (15) for attaching the stimulation current unit (14).

4. A device as claimed in any one of claims 1 to 3, characterized in that the opened sleeve (1) is substantially T-shaped.

5. A device as claimed in claim 4, characterized in that the sleeve (1) has in the centre of its wider portion resulting from the T-shape a trough-like depression (17) extending in the longitudinal direction of the said sleeve.

6. A device as claimed in any one of claims 1 to 5, characterized in that the connection leads (13) from the stimulation current unit (14) to the electrodes (7 to 12) are disposed so as to extend in the interior of the sleeve (1).

## Revendications

1. Dispositif pour le traitement électrique de parties du corps, se composant d'une pièce d'ajustage qui est fermée hermétiquement, sur une ouverture au moins et en outre peut être adaptée à la partie du corps à traiter, dans lequel la paroi intérieure de la pièce d'ajustage est flexible et porte au moins deux électodes (7, 10) de grande surface, constituées de matériau conducteur et réalisées en feuilles qui, sous pression, s'appliquent hermétiquement à la partie du corps à traiter et peuvent être connectées par des lignes de raccordement à un appareil à courant d'excitation, caractérisé en ce que la pièce d'ajustage est exécutée comme une manchette (1) à couble paroi gonflable et flexible et peut être branchée à un raccord (3) sur une pompe (6) et en ce que les deux électrodes (7, 10) et au moins deux autres électrodes (8, 11 ou 9, 12) sont disposées l'une près de l'autre dans le sens perpendiculaire à la direction longitudinale de la manchette (1), à intervalle.

2. Dispositif selon la revendication 1, caractérisé en ce que la manchette (1) présente, dans le sens de sa longueur, une fermeture-éclair (2) ou un ruban auto-accrochant.

3. Dispositif selon les revendications 1 ou 2, caractérisé en ce que la manchette (1) présente sur sa paroi extérieure un support (15) pour fixer l'appareil à courant d'excitation (14).

4. Dispositif selon les revendications 1 à 3, caractérisé en ce que la manchette (1) ouverte a, à peu près, la form d'un T.

5. Dispositif selon la revendication 4, caractérisé en ce que la manchette (1) présente au milieu de sa plus large section donnée par la forme en T, un creux (17) à la façon d'une auge, se développant dans le sens de sa longueur.

6. Dispositif selon les revendications 1 à 5, caractérisé en ce que les conducteurs de raccordement (13) de l'appareil à courant d'excitation (14) aux électrodes (7 à 12) sont disposés avec passage à l'intérieur de la manchette (1).

Fig.1

Fig.2

EP 0 211 146 B1

Fig.3

Fig.4